# EUROPÄISCHE PATENTANMELDUNG

(11) **EP 3 643 758 A1**
(43) Veröffentlichungstag der Anmeldung: **29.04.2020**
(21) Anmeldenummer: 18202647.6
(22) Anmeldetag: 25.10.2018
(51) Int. Cl.: C09D 127/16, A61L 27/34, A61N 1/05, C08F 283/12, C09D 127/18, C09D 133/06, C09D 171/12, C09D 177/00, C09D 181/06

(54) **SILIKONWERKSTOFF MIT MODIFIZIERTER OBERFLÄCHE ZUR VERBESSERUNG DER GLEIT- UND REIBEIGENSCHAFTEN**

(71) Anmelder: BIOTRONIK SE & Co. KG, 12359 Berlin (DE)
(72) Erfinder: Bock, Ralf, 12049 Berlin (DE); Borck, Alexander, 15754 Heidsesee (DE)
(74) Vertreter: Keck, Hans-Georg

(57) **Zusammenfassung**

Die vorliegende Erfindung betrifft einen Werkstoff umfassend ein Silikon, wobei ein Polymer an der Oberfläche des Silikons angeordnet ist, das Polymer durch eine höhere Abriebfestigkeit als das Silikon charakterisiert ist, und das Polymer über nicht-kovalente Bindungen an der Oberfläche des Silikons befestigt ist.

## Beschreibung

Silikone oder genauer Poly(organo)siloxane stellen eine der wichtigsten Stoffklassen in der Medizintechnik dar. Dennoch werden Anstrengungen unternommen, neue Materialien einzuführen, da Silikon neben vielen Vorteilen auch Nachteile aufweist. Einen wesentlichen Nachteil stellt die vergleichsweise geringe Durchriebfestigkeit dar. Silikon ist ein dreidimensional vernetztes Polymer. Die gewünschte Härte wird zum Teil über die Netzwerkdichte eingestellt, vor allem aber über Zuschlagstoffe wie Quarzmehl. Diese Zuschlagstoffe sind zum Teil dafür verantwortlich das die Materialien so nachteilige Reibeigenschaften aufweisen. Diese Zuschlagsstoffe können bei mechanischer Belastung aus der Matrix austreten und dann als abrasive Substanz wirken.

Bekannte Lösungen sehen beispielsweise vor, dass das Silikon mit Außenschläuchen aus einem thermoplastischen Polyurethan (TPU) überzogen wird oder das Silikon komplett durch ein TPU ersetzt wird. Die Außen- bzw. Coverschläuche müssen jedoch in einem separaten, anspruchsvollen Prozess mit zum Teil sehr geringen Wandstärken gefertigt und dann aufmontiert werden.

Nachteilig an allen thermoplastischen Polyurethanen (TPU) ist ihre mangelnde Hydrolyse- und Biostabilität, insbesondere aufgrund der nicht hydrolysenstabilen Polyurethanbindung. Insbesondere die Metallionenoxidation führt zu einem vorzeitigen Abbau des Isolationsmaterials. Ferner zeigen TPU basierend auf aromatischen Isocyanaten große Schwächen bei der Biokompatibilität. Um die Hydrolyseanfälligkeit zu minimieren, wurden Anstrengungen unternommen, sehr lipophile TPU Rezepturen zu verwenden. Damit soll die Wasseraufnahme und entsprechend die Hydrolyse minimiert werden. Ein weiterer Nachteil ist die hohe Gleitreibung und Haftreibung. Weiterhin zeigen weiche TPU Materialien ein klebriges Oberflächenverhalten. Zusätzlich ist bei Verwendung eines TPU Grundkörpers eine Beschichtung unerlässlich.

Somit weist TPU als Alternative zu Silikon beträchtliche Nachteile auf, insbesondere wenn TPU als Isolierungsmaterial für Elektroden bzw. Elektrodenleitungen verwendet wird.

Basierend auf diesem Hintergrund ist es daher eine Aufgabe der vorliegenden Erfindung, einen geeigneten Werkstoff zur Verfügung zu stellen, beispielsweise als Isolierungsmaterial für Elektroden bzw. Elektrodenleitungen von medizinischen Implantaten, der eine höhere Abriebfestigkeit und/oder geringere Gleitreibung oder Haftreibung als beispielsweise Silikon oder TPU aufweist.

Diese Aufgabe wird durch einen Werkstoff mit den Merkmalen des Anspruchs 1 sowie durch ein Verfahren mit den Merkmalen des Anspruches 10 gelöst. Vorteilhafte Ausgestaltungen sind in den abhängigen Ansprüchen sowie im Folgenden beschrieben.

Demnach wird ein Werkstoff gemäß Anspruch 1 zur Verfügung gestellt. Der erfindungsgemäße Werkstoff umfasst ein Silikon, wobei an der Oberfläche des Silikons ein Polymer angeordnet ist, welches durch eine höhere Abriebfestigkeit als Silikon charakterisiert ist, und das Polymer durch nicht-kovalente Bindungen an der Oberfläche des Silikons befestigt ist.

Insbesondere ist das Polymer ausschließlich durch nicht-kovalente Bindungen an der Oberfläche des Silikons befestigt.

Als Silikone im Sinne der Erfindung werden insbesondere Polysiloxane mit der allgemeinen Formel [R₂SiO]ₙ bezeichnet, wobei R eine Kohlenstoffverbindung ist, insbesondere ein Alkyl, wie etwa Methyl oder Ethyl, oder ein Aryl wie etwa Phenyl.

Als Abriebfestigkeit wird im Sinne der Erfindung die Widerstandsfähigkeit einer festen Oberfläche gegenüber mechanischer Beanspruchung, insbesondere Reibung, bezeichnet.

Der Begriff "Anordnen des Polymers an die Oberfläche des Silikon" im Sinne der Erfindung bedeutet insbesondere, dass das Polymer nicht-kovalent bzw. physikalisch an der Silikonoberfläche befestigt ist. Vorzugsweise wird das Polymer durch nicht-kovalente Interaktionen wie elektrostatische oder hydrophobe Wechselwirkungen, Wasserstoffbrücken oder Van-der-Waals-Kräfte, oder durch Verschlaufung der Ketten des Silikons mit den Ketten des Polymers, an der Oberfläche angeordnet bzw. physikalisch befestigt.

Insbesondere ist die Oberfläche des Silikons mit dem Polymer beschichtet.

Diese erfindungsmäßige Lösung liefert ein Silikon, das nicht nur gute Reibwerte aufweist, sondern auch extrem durchriebfest ist. Durch die verfahrenstechnisch wenig aufwendige Oberflächenbeschichtung bzw. Befestigung des Polymers an der Oberfläche des Silikons entfallen, beispielsweise die Coverschlauchherstellung und auch die Montage des Coverschlauches auf dem Grundkörper von Elektrodenleitungen von implantierbaren Geräten. Das, kombiniert mit den exzellenten Eigenschaften des Silikons, macht die Materialkombination bzw. den Werkstoff zu einer verbesserten Alternative zu herkömmlichen Materialien.

Gemäß einer Ausführungsform des erfindungsgemäßen Werkstoffes ist vorgesehen, dass das Polymer ein Copolymer aus zwei oder mehreren Monomeren ist.

Gemäß einer weiteren Ausführungsform des erfindungsgemäßen Werkstoffes ist vorgesehen, dass ein oder mehrere Monomere des Polymers aus der Gruppe ausgewählt sind, die
- ein aromatisches Diphenol, insbesondere Bisphenol A oder 1,4-Dihydroxybenzen, und ein Bus(halophenyl)sulfon, insbesondere 4,4-Dichlordiphenylsulfon,
- Vinylidenfluorid, und
- ein Acrylat, ein Methacrylat (MAA) und/oder ein Methylmethacrylat (MMA),
umfasst.

Gemäß einer weiteren Ausführungsform des erfindungsgemäßen Werkstoffes ist vorgesehen, dass das Polymer 2-Methacryloyloxyethylphosphorycholin (CAS Nr.: 67881-98-5) als Monomer umfasst, vorzugsweise in Kombination mit Acrylat, Methacrylat und/oder Methylmethacrylat.

Gemäß einer weiteren Ausführungsform des erfindungsgemäßen Werkstoffes ist vorgesehen, dass das Polymer ein Polyvinylidenfluorid, ein Polyarylsulfon, insbesondere ein Polysulfon, ein Polyacrylat, ein Polymethylacrylat, oder ein Polymethylmethacrylat ist.

Gemäß einer weiteren Ausführungsform des erfindungsgemäßen Werkstoffes ist vorgesehen, dass das Polymer ein Polysulfon (CAS Nr. 25135-51-7), ein Polyethersulfon (CAS Nr. 25608-63-3) oder ein Polyphenylensulfon (CAS Nr. 25608-64-4) ist.

Gemäß einer weiteren Ausführungsform des erfindungsgemäßen Werkstoffes ist vorgesehen, dass das Polymer ein Polytetrafluorethylen (PTFE), Ethylen-Tetrafluorethylen (ETFE), Polyamid, Polyetheretherketon (PEEK) ist.

Gemäß einer weiteren Ausführungsform des erfindungsgemäßen Werkstoffes ist vorgesehen, dass das Polymer über Verschlaufung der Ketten des Silikons und der Ketten des Polymers an der Oberfläche des Silikons befestigt ist.

Als Verschlaufung im Sinne der vorliegenden Erfindung wird insbesondere eine physikalische/geometrische Verbindung von zwei oder mehr Polymerketten bezeichnet, die entsteht, indem sich die Polymerketten überkreuzen. Durch die Verschlaufung lässt sich die eine Polymerkette durch Krafteinwirkung nur erschwert oder gar nicht von der anderen Polymerkette oder den anderen Polymerketten trennen.

Gemäß einer weiteren Ausführungsform des erfindungsgemäßen Werkstoffes ist vorgesehen, dass das Silikon ein Methyl-Silikon, insbesondere ein Polydimethylsiloxan, ein Vinyl-Methyl-Silikon, ein Phenyl-Vinyl-Methyl-Silikon, ein phenylmodifiziertes Silikon, ein Fluoroalkyl-Silikon oder ein Fluor-Vinyl-Methyl-Silikon ist.

Gemäß einer weiteren Ausführungsform des erfindungsgemäßen Werkstoffes ist vorgesehen, dass das Polymer ein Copolymer aus
a. einem Acrylat, einem Methacrylat und/oder einem Methylmethacrylat; und
b. einer zwitterionischen Verbindung, ausgewählt aus Phosphorylcholin, Sulfobetain, Carboxybetain, Sulfopyridiumbetain, Cystein, oder ein Sulfobetainsiloxan, vorzugsweise umfassend Methyl- oder Ethylgruppen, ist.

Vorzugsweise weist die zwitterionische Verbindung mindestens einen polymerisierbaren Rest wie etwa eine Vinylgruppe oder eine Acrylgruppe auf.

Gemäß einem weiteren Aspekt der Erfindung wird ein medizinisches Implantat zur Verfügung gestellt, wobei das medizinische Implantat den erfindungsgemäßen Werkstoff aufweist.

Gemäß einer Ausführungsform des erfindungsgemäßen medizinischen Implantats ist vorgesehen, dass das medizinische Implantat eine Elektrodenleitung umfasst.

Als Elektrodenleitung wird im Sinne der Erfindung insbesondere eine Vorrichtung bezeichnet, die mindestens ein langgestrecktes elektrisch leitfähiges Element, insbesondere einen Draht umfasst, welches sich über die Länge der Elektrodenleitung erstreckt. Insbesondere umfasst die Elektrodenleitung eine elektrische, insbesondere röhrenförmige, Isolierung, wobei das Lumen der Isolierung das mindestens eine langgestreckte Element mit Ausnahme der beiden Enden umfasst. Eine solche Isolierung wird auch als Coverschlauch bezeichnet.

Gemäß einer weiteren Ausführungsform des medizinischen Implantats ist vorgesehen, dass die Elektrodenleitung, insbesondere das langgestreckte leitfähige Element, zumindest zum Teil mit dem erfindungsgemäßen Werkstoff ummantelt ist.

Gemäß einer weiteren Ausführungsform des medizinischen Implantats ist vorgesehen, dass das medizinische Implantat eine stromerzeugende bzw. stromgebende Komponente (Generatorkomponente, Batterie) und/oder eine stromerfassende Komponente (Diagnosekomponente) umfasst, welche mit der Elektrodenleitung verbunden ist.

Gemäß einer weiteren Ausführungsform des medizinischen Implantats ist vorgesehen, dass das medizinische Implantat als ein Herzschrittmacher, ein Kardioverter-Defibrillator oder ein Neurostimulator, insbesondere ein Rückenmarksstimulator, ausgebildet ist.

Bei einem Herzschrittmacher handelt es sich insbesondere um eine Vorrichtung, die einen implantierbaren elektronischen Impulsgenerator umfasst, der über eine Elektrodenleitung mit dem Herzen eines Patienten verbunden ist.

Bei einem implantierbaren Kardioverter-Defibrillator (ICD) handelt es sich insbesondere um eine Vorrichtung, die einen Stimulationsteil (Impulsgenerator) und einen Diagnostikteil (zur Erkennung bedrohlicher Rhythmusstörungen) aufweist, wobei der Stimulations/Diagnostikteil, meist unter der Haut in der Nähe des linken Brustmuskels implantiert, über eine Elektrodenleitung mit der rechten Herzkammer verbunden wird. Die Elektrodenleitung wird hierbei typischerweise über die obere Hohlvene in die rechte Herzkammer geführt.

Bei dem Rückenmarksstimulator handelt es sich insbesondere um eine Vorrichtung zur Behandlung von chronischen neuropathischen Schmerzen, in der ein Impulsgenerator über eine Elektrodenleitung mit dem zu behandelnden Teil des Nervensystems verbunden ist, beispielsweise mit dem Hinterstrang des Rückenmarks.

Gemäß einer weiteren Ausführungsform des medizinischen Implantats ist vorgesehen, dass das medizinische Implantat eine Fixierhülse zum Fixieren einer implantierbaren Elektrodenleitung (Elektrodenfixierhülse, EFH) umfasst, wobei die Elektrodenfixierhülse den erfindungsgemäßen Werkstoff umfasst oder im Wesentlichen daraus besteht.

Als Elektrodenfixierhülse wird im Sinne der Erfindung insbesondere eine spezielle Fixierhülse bezeichnet, die an einer definierten Stelle fest auf der Elektrodenleitung angeordnet wird und die dann an geeigneter Stelle im menschlichen Körper befestigt wird, zum Beispiel durch Annähen im Bereich eines Muskels oder eines Gefäßes.

Gemäß eines weiteren Aspekts der Erfindung wird ein Verfahren zur Herstellung des erfindungsgemäßen Werkstoffes zur Verfügung gestellt. Das Verfahren umfasst die Schritte:
- Bereitstellen eines Silikons, und
- Anordnen eines Polymers auf der Oberfläche des Silikons, wobei das Polymer durch eine höhere Abriebfestigkeit als das Silikon gekennzeichnet ist, und das Polymer durch nicht-kovalente Bindungen an der Oberfläche des Silikons befestigt wird.

Bei dem Silikon und dem Polymer handelt es sich jeweils um ein Silikon und ein Polymer gemäß einem der oben stehenden Aspekte oder Ausführungsformen.

Der Begriff "Anordnen eines Polymers" im Sinne der Erfindung bedeutet insbesondere das physikalische Befestigen des Polymers an der Oberfläche des Silikons über nicht-kovalente Bindungen. Insbesondere wird die Oberfläche des Silikons zumindest zum Teil mit dem Polymer beschichtet.

Gemäß einer Ausführungsform des erfindungsgemäßen Verfahrens ist vorgesehen, dass das Anordnen des Polymers auf die Oberfläche des Silikons folgende Schritte umfasst:
- Lösen des Polymers in einem geeigneten Lösungsmittel unter Erhalt einer Polymerlösung;
- Auftragen der Polymerlösung auf das Silikon, vorzugsweise durch Aufsprühen oder Eintauchen; und
- Trocknen des Silikons bei einer Temperatur im Bereich von Raumtemperatur bis etwa 300 °C, vorzugsweise bei Temperaturen von 180°C bis 260 °C und weiter bevorzugt bei etwa 220 °C.

Gemäß einer weiteren Ausführungsform des erfindungsgemäßen Verfahrens ist vorgesehen, dass das Anordnen des Polymers auf die Oberfläche des Silikons folgende Schritte umfasst:
- Quellen des Silikons in einem organischen Lösungsmittel unter Erhalt eines gequollenen Silikons,
- In Kontakt bringen des gequollenen Silikons mit dem Polymer, wobei das Polymer gelöst in einem geeigneten Lösungsmittel vorliegt, und
- Trocknen des Silikons.

Alternativ umfasst das Anordnen des Polymers auf die Oberfläche des Silikons folgende Schritte:
- Quellen des Silikons in einem organischen Lösungsmittel unter Erhalt eines gequollenen Silikons;
- In Kontakt bringen des gequollenen Silikons mit einem oder mehreren Vorläufern des Polymers, wobei der eine Vorläufer des Polymers gelöst in einem geeignetem Lösungsmittel (inerter Reaktionspartner) vorliegt oder ein Vorläufer des Polymers in einem anderen Vorläufer des Polymers (reaktiver Reaktionspartner) gelöst vorliegt;
- Umsetzen bzw. Polymerisieren des einen oder der mehreren Vorläufer zum Polymer; und
- Trocknen des Silikons.

Unter Quellen des Silikons wird im Sinne der Erfindung insbesondere das Eindringen des organischen Lösungsmittels in das Silikon verstanden, wobei zumindest die obersten Schichten des Silikons eine Volumenvergrößerung erfahren. Das Quellen des Silikons bewirkt insbesondere, dass die Silikonketten der obersten Schichten beweglicher werden und somit vorteilhafterweise mit den Polymerketten des Polymers Verschlaufungen ausbilden können.

Ebenso ermöglicht das Quellen des Silikons das Polymer innerhalb der beweglichen Ketten des Silikons sich aus Monomeren durch polymerisieren aufbauen, wodurch auch hier vorteilhafterweise eine Verschlaufung der Ketten des Polymers und des Silikons erzielt werden kann.

Ein wesentlicher Vorteil des erfindungsgemäßen Verfahrens ist es, dass das Verfahren in jedem Schritt eines Herstellungsverfahrens zur Herstellung einer Vorrichtung oder eines Geräts durchgeführt werden kann, der den erfindungsgemäßen Werkstoff umfassen soll. Beispielsweise können bereits vorhandene Elektrodenleitungen mit handelsüblichen Silikonummantelungen mit dem erfindungsgemäßen Verfahren behandelt werden, um so Elektrodenleitungen mit dem erfindungsgemäßen Werkstoff zu erhalten.

Trocknen des Silikons im Sinne der Erfindung bedeutet insbesondere das Entfernen des organischen Lösungsmittels, insbesondere das technisch mögliche vollständige Entfernen des organischen Lösungsmittels, durch beispielsweise Temperatur oder Anlegen eines Vakuums.

Zum Umsetzen bzw. Polymerisieren des einen oder der mehreren Vorläufer zum Polymer können Initiatoren verwendet werden, wie beispielsweise Azobis(isobutyronitril) (AIBN) als Initiator einer Radikal-Kettenreaktion verwendet werden, beispielsweise bei der Polymerisation von Acrylat, Methacrylat (MAA), und/oder Methylmethacrylat (MMA).

Gemäß einer weiteren Ausführungsform des erfindungsgemäßen Verfahrens ist vorgesehen, dass der oder die Vorläufer Monomere sind, die aus der folgenden Gruppe ausgewählt sind:
- ein aromatisches Diphenol, insbesondere Bisphenol A oder 1,4-Dihydroxybenzen, und ein Bis(halophenyl)sulfon, insbesondere 4,4-Dichlordiphenylsulfon,
- Vinylidenfluorid,
- ein Acrylat, ein Methacrylat (MAA), und/oder ein Methylmethacrylat (MMA).

Gemäß einer weiteren Ausführungsform des erfindungsgemäßen Verfahrens ist vorgesehen, dass die Vorläufer
a. Acrlyat, Methacrylat und/oder Methacrylatmethylester; und
b. eine zwitterionische Verbindung ausgewählt aus Phosphorylcholin, Sulfobetain, Carboxybetain, Sulfopyridiumbetain, Cystein, Phosphatidylethanolamin, oder ein Sulfobetainsiloxan, vorzugsweise umfassend Methyl- oder Ethylgruppen,
umfassen, wobei die zwitterionische Verbindung vorzugsweise eine polymerisierbare Gruppe wie etwa eine Vinylgruppe oder eine Acrylgruppe aufweist.

Vorzugsweise lassen sich mit den oben beschriebenen zwitterionischen Verbindungen als Copolymer antimikrobielle Oberflächen herstellen.

Vorzugsweise umfassen die Vorläufer Methacrylat, Methylmethylacrylat und Phosphorylcholin bzw. 2-Methacryloyloxyethylphosphorylcholin.

Gemäß einer weiteren Ausführungsform des erfindungsgemäßen Verfahrens ist vorgesehen, dass das organische Lösungsmittel Toluol, Xylol, Chloroform, Dichlormethan, Dimethylacetamind, Dimethylformamid, Dimethylsulfoxid, Kresol, -ortho-Dichlorbenzol, Sulfolan, Tetrahydrofuran, Trichlorethylen, N-Methyl-2-pyrrolidon, oder eine Methacrylat- oder Methylmethacrylatlösung ist. Ein bevorzugtes Lösungsmittel zum Quellen des Silikons ist N-Methyl-2-pyrrolidon, insbesondere in Kombination mit n-Heptan.

Weitere Merkmale und Ausführungsformen der vorliegenden Erfindung sollen nachfolgend anhand der Figuren erläutert werden. Es zeigen:
- Fig. 1: geeignete Polymere zur Verwendung im erfindungsgemäßen Werkstoff;
- Fig. 2: schematisch ein mögliches Verfahren zur Herstellung des erfindungsgemäßen Werkstoffes;
- Fig. 3: eine Ausführungsform eines erfindungsgemäßen Implantats, aufweisend eine Elektrodenleitung;
- Fig. 4: eine weitere Ausführungsform eines erfindungsgemäßen medizinischen Implantats, aufweisend eine Elektrodenfixierhülse; und
- Fig. 5: die Ergebnisse von Bewuchsversuchen aus Zellkulturversuchen auf verschiedenen Materialien.

### Beispiele

Die vorliegende Erfindung stellt insbesondere ein Isolationsmaterial zur Verfügung, welches die bekannten positiven Eigenschaften von Silikon zeigt, aber darüber hinaus die bekannten Nachteile nicht mehr aufweist. Hier werden vor allem Probleme des Ab- bzw. Durchriebs reduziert bzw. vermieden. Sowohl die Abriebphänomene Lead-to-Lead als auch die Durchriebeigenschaften Inside-Outside können so umgangen bzw. ausgeschlossen werden. Insgesamt zeigen die hergestellten erfindungsgemäßen Materialien bzw. Werkstoffe eine hohe Stabilität gegenüber Reibeinflüssen auf.

Vorteilhafterweise können durch die erfindungsgemäßen Gegenstände beispielsweise bei der Herstellung einer Elektrodenleitung zwei Produktionsschritte eingespart werden. Die Herstellung eines maßhaltigen Coverschlauches und die Montage desselben werden durch das erfindungsgemäße Verfahren abgelöst. Vorteilhafterweise können handelsübliche bzw. bereits bewährte Elektrodenleitungsdesigns durch das erfindungsgemäße Verfahren verbessert bzw. auf die Anforderungen im Markt angepasst werden. Insbesondere werden die bekannten großen Probleme der implantierbaren Elektrodenleitungen, Durchrieb und Abrieb, gelöst, wobei die Handhabungseigenschaften nicht verschlechtert werden.

Dafür wird insbesondere Silikon mit geeigneten Materialien beschichtet. Dabei ist die Beschichtung dauerhaft und nicht abrasiv. Abgesehen von den Gleit-und Reibeigenschaften, werden die mechanischen Eigenschaften durch die Beschichtung vorteilhafterweise nicht beeinflusst. Die derart modifizierte Oberfläche des Silikons erlaubt es, daraus hergestellte Vorrichtungen bzw. Geräte als Dauerimplantate einzusetzen.

Insbesondere kann die Silikonoberfläche so modifiziert werden, dass eine Gewebsanhaftung nach Implantation vermindert wird. Eine aus diesem Material hergestellte Elektrodenleitung kann sich auch nach Jahren aus dem Körper ohne Schwierigkeiten entfernen lassen. Ein weiterer Punkt ist die antimikrobielle Beschichtung. Synergien zwischen den beiden Stoßrichtungen könnten vorliegen. Das heißt, eine geringe, unspezifische Proteinadsorption kann auch einen Vorteil bei der Implantat assoziierten Infektion spielen.

Silikonkautschuk, im folgendem einfach mit Silikon bezeichnet, bietet kaum reaktive Gruppen, die eine chemische Modifikation möglich machen. Silikon quillt in einigen organischen Lösungsmitteln wie Toluol oder Xylol auf. In dieser gequollenen Matrix kann ein Monomer eindiffundieren, welches dann durch radikalische Polymerisation zu Polymerketten aufgebaut wird. Nach dem Entfernen des Quellungsmittels werden diese neu gebildeten Ketten dann mechanisch in der Matrix eingeklemmt. Zum anderen sollen geeignete Polymere nach der Quellung eindiffundieren. Nach dem Entfernen des Lösungsmittels sind diese dann auch an und auf der Oberfläche fixiert.

### Bestimmung des Quellungsverhaltens von Silikon in verschiedenen Lösungsmitteln

Die Silikonproben wurden gewogen und für 24 h in das zu untersuchende Lösungsmittel eingelegt. Nach der Quellung werden die Scheibchen oberflächlich abgetupft und erneut gewogen.

Der Quellungsfaktor wird nach folgender Formel Q/100=(a-b)/b) berechnet:
Dabei ist Q der Quellungsfaktor und a das Gewicht nach Quellung und b das ursprüngliche Gewicht. Die Ergebnisse sind in der folgenden Tabelle gezeigt.

**Tabelle 1: Quellungsfaktoren von Silikon in unterschiedlichen Lösungsmitteln**

| DMF | Toluol | Xylol | Wasser | Methanol | DMSO | MMA |
|---|---|---|---|---|---|---|
| 3 | 32 | 86 | 1 | 2 | 3 | 60 |

### Testpolymerisationen mit MMA:

i) Die Silikonscheiben wurden 24 h bei Raumtemperatur in Toluol vorgequollen. Dann wurden sie in die drei unterschiedlich konzentrierten Polymerisationslösungen gegeben. Die Lösungen wurden vorher mit N₂ entgast. Die jeweilige Polymerisationslösung bestand aus 40 ml Wasser mit a)1 ml MMA; b)2,5 ml MMA und c)5 ml MMA. Die Reaktion wurde mit 2,2'-Azobis(2-methylpropionamidin)dihydrochlorid bei 80 °C gestartet.
ii) Alternativ wurden die Silikonproben in MMA gequollen und danach in die unter i) beschriebenen Polymerisationslösungen gegeben.
iii) Quellung in DMF als relativ schlechtes Quellungsmittel über 24 h. Dann Polymerisation in 40 ml Wasser mit 5 ml MMA. Das MMA sollte das DMF verdrängen, weil die Affinität des MMA zu Silikon weitaus höher ist als zum Lösungsmittel Wasser. Entsprechend Ansatz i).

Alle Proben wurden bei 80 °C im Vakuum getrocknet.

### Beschichtung der Außenfläche einer Elektrodenleitung aus Silikon mit PVDF oder PSU.

Das bekanntermaßen biokompatible und durchriebstabile PVDF kann über unterschiedliche Verfahren aufgebracht werden.

Zum einen kann ein Beschichtungsverfahren mit einem Einbrennvorgang verwendet werden. Dazu wird PVDF in einem geeigneten Lösungsmittel gelöst, aufgesprüht oder getaucht. Der Einbrennvorgang bei Temperaturen von 240 °C stellt für die Silikonmatrix kein Problem dar. Eine Vorbehandlung des Silikons mit einem Primer oder Haftvermittler ist machbar, aber nicht zwingend erforderlich. Die Reinigung des Silikons vor dem aufbringen des PVDF's ist zwingend notwendig. Die Reinigungsverfahren sind dem Fachmann bekannt.

Ein weiterer geeigneter Weg um PVDF auf Silikonkautschuk zu fixieren ist mit Hilfe von Lösungsmitteln realisierbar, die das Silikon zum Quellen bringen. Das Silikon wird mit Cyclohexan oder Cycloheptan, allgemein einem Kohlenwasserstoff, gequollen. Zu dieser aufgequollenen Matrix wird jetzt PVDF in einem geeigneten Lösungsmittel gegeben. Die Elektrodenleitung wird aus der Beschichtungslösung entnommen und getrocknet. Dabei kommt es zu einer Entquellung. PVDF ist durch Verschlaufungsmechanismen bürstenförmig auf der Silikonoberfläche immobilisiert. Eine Vorbehandlung des Silikons mit einem Primer oder Haftvermittler ist machbar aber nicht zwingend erforderlich.

Neben dem bevorzugten PVDF ist auch Polysulfon oder Polyethersulfon als Beschichtungsmaterial geeignet. Aus hier geschieht die Beschichtung aus einem Lösungsmittel mit dem "Kunstgriff" der Verwendung von Kohlenwasserstoffen um die reversible Quellung von Silikon zu nutzen. Ebenfalls, wie oben, wird ausgenutzt, dass sich die Silikonmatrix ausweitet und so sich die Beschichtungspolymere in die Matrix einbauen können.

Durch die Materialkombination lässt sich auch ein "altes" Elektodendesign verwenden, das durch die Oberflächenmodifikation ein attraktives Verkaufsargument erhält. Im Gegensatz zu Beschlauchungsverfahren, ist ein simples Tauchverfahren kostengünstiger. Es lassen sich so Elektrodenleitungen mit vergleichbaren Oberflächeneigenschaften kostengünstiger herstellen. Die Gleiteigenschaften, wie auch die Reibeigenschaften werden im Vergleich zum Silikon verbessert. Beschichtungen mit PVDF aber auch von Polysulfon oder Polyethersulfon sind auch TPU Oberflächen überlegen. Insbesondere deshalb, weil alle TPU Materialien einem hydrolytischen Abbau unterworfen sind.

Polysulfon (PSU) wird als Trennmembran für Dialysemembranen verwendet. Das Polymer zeigt ebenfalls eine nur geringe Proteinadsorptionsneigung. Ebenfalls wird es als Membranmaterial in der Biotechnologie verwendet, wenn insbesondere proteinhaltige Lösungen gefiltert werden sollen, diese aber nicht am Filter verbleiben sollen.
In Experimenten wurde PSU im Tierversuch mit großem Erfolg getestet. Hier konnte auch nach 4 Monaten keine Protein-, respektive Zellanhaftung beobachtet werden.

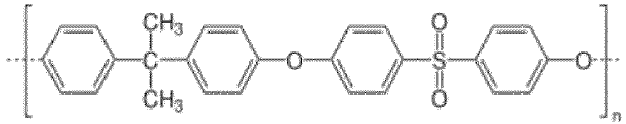

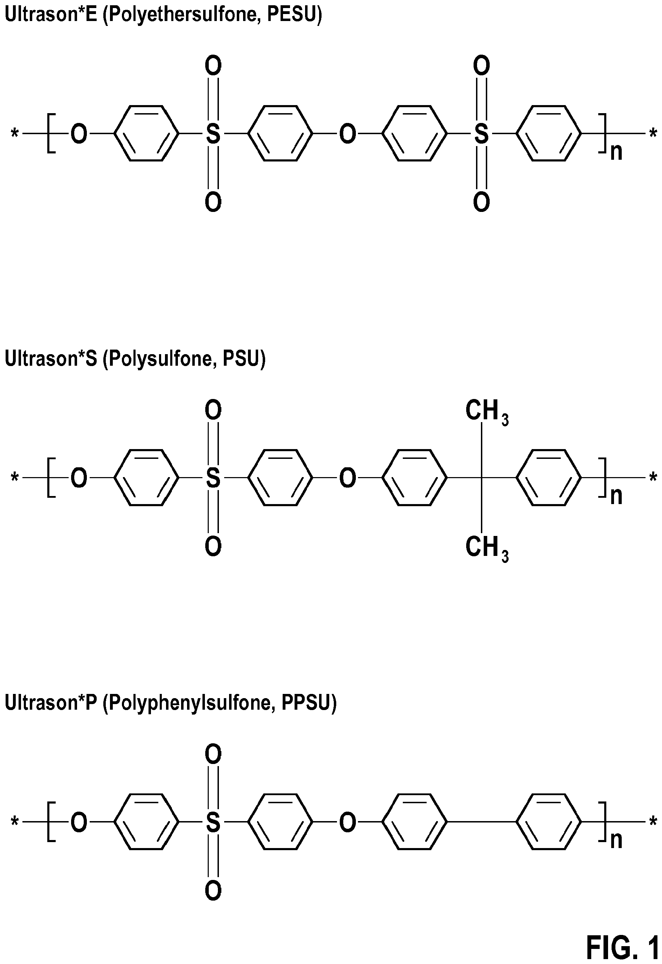

Als besonders geeignet für die oberflächendurchdringende Beschichtung gemäß der Erfindungsmäßigen Lösung sind die folgenden Polymere aus der Klasse der Polysulfone. In Figur 1 werden die Handelsnamen der BASF benutzt.

Als Lösungsmittel kommen: Chloroform, Dichlormethan, Dimethylacetamid und N-Methyl-2-pyrrolidon in Frage, wobei N-Methyl-2-pyrrolidon bevorzugt wird. N-Methyl-2-pyrrolidon kann gut mit Lösungsmitteln wie n-Heptan kombiniert werden um die gewünschte Quellung des Silikons zu erreichen.

Weitere geeignete Lösungsmittel sind Toluol, Dichlormethan, Dimethylacetamid, Dimethylformamid, Dimethylsulfoxid, Kresol, ortho-Dichlorbenzol, Sulfolan, Tetrahydrofuran, Trichlorethylen.

### Beschichtung einer Silikonelektrodenleitung mit PSU

Die Beschichtung unterscheidet sich von herkömmlichen Beschichtungen dadurch, dass keine Kohäsionschicht vorhanden ist, sondern, dass das Beschichtungsmaterial auch die Oberfläche durchdringt und bis in das Innere des Silikons reicht.

Das Silikonmaterial wird mit Hilfe von n-Heptan zum Aufquellen gebracht. Das geschieht bei Raumtemperatur für 1 min bis 24 h. Über die Zeit kann der Umfang der Quellung beeinflusst werden und somit auch die Tiefe, in der das Polysulfon in das Innere des Trägermaterials (Silikonkautschuk) eindringt. Vorzugsweise wird nur eine Quellzeit von 15 bis 45 min genutzt.
Danach wird das gequollene Material durch eine 5 % Polysulfonlösung in N-Methyl-2-pyrrolidon gezogen.

Zur Herstellung der Polysulfonlösung wird das Polysulfon eingewogen und mit dem Lösungsmittel aufgefüllt. Im Anschluss wird die Lösung für 24 h bei 80 °C gerührt.
Die Einwirkzeit der Lösung als das Trägermaterial beträgt 1 bis 120 min. Vorzugsweise wird das Silikon für 2 bis 20 min in der Polymerlösung belassen.

Das Lösungsmittel wird durch Verdampfen bei Raumtemperatur über 3 bis 4 Tage entfernt. Darauf werden Lösungsmittelreste durch eine Vakuumbehandlung bei 80 °C für 7 Tage restlos entfernt.

### Herstellung von Silikonkautschuk mit MMA/MAA/PC Modifikation:

Das folgende Ausführungsbeispiel beschreibt die Oberflächenmodifikation einer Silikonkautschuk Matrix mit Methylmetarylat (MMA)/Methacrylsäure (MAA) und einem zwitterionischem Molekül.

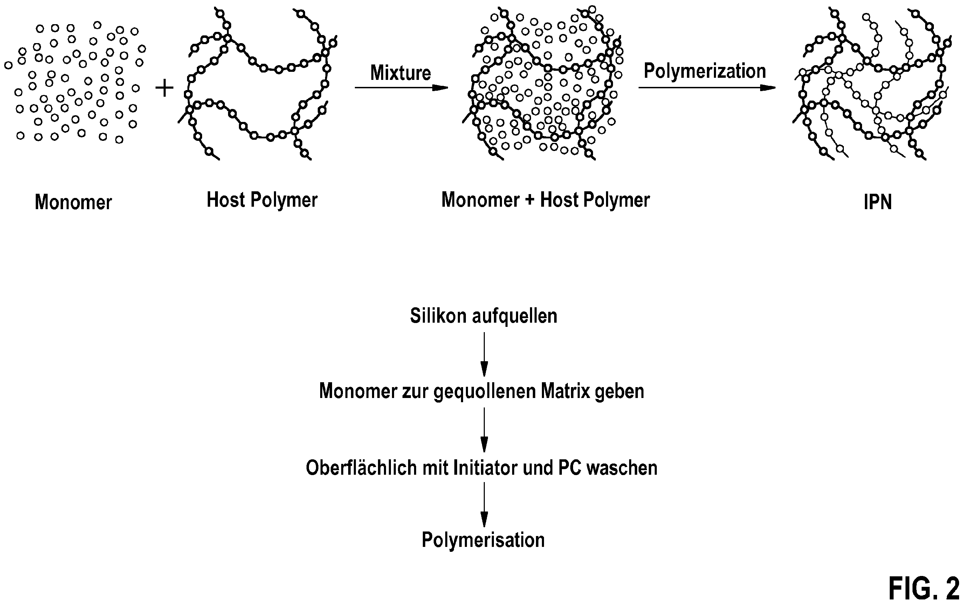

Dabei wird die Silikonmatrix mit einem Lösungsmittel zum Quellen gebracht. Durch die Quellung steht ein Volumen zur Verfügung, in dem Monomere zu Präpolymeren und Polymeren aufgebaut werden können. Das in Figur 2 oben dargestellte Schema verdeutlicht den Aufbau dieses Interpenetrationsnetzwerkes (IP-Netzwerk).

Als Zwitterion sind eine Reihe von Verbindungen anwendbar. Im folgenden Schema sind einige Zwitterionen aufgeführt.

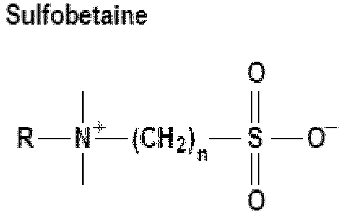

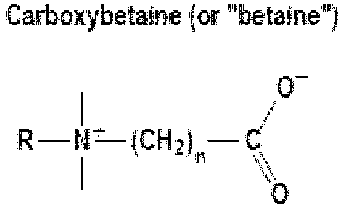

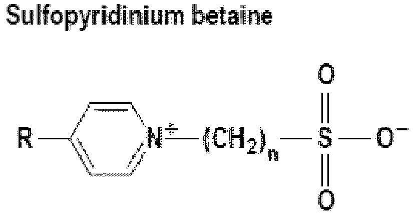

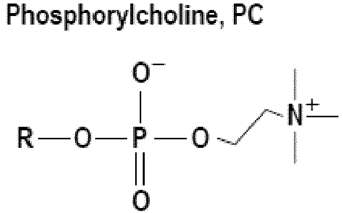

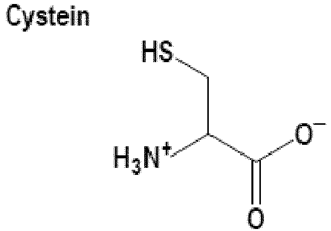

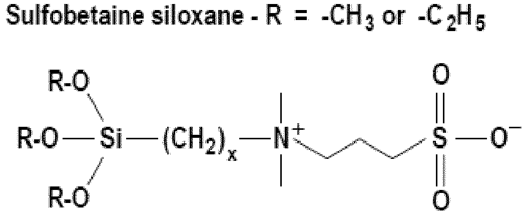

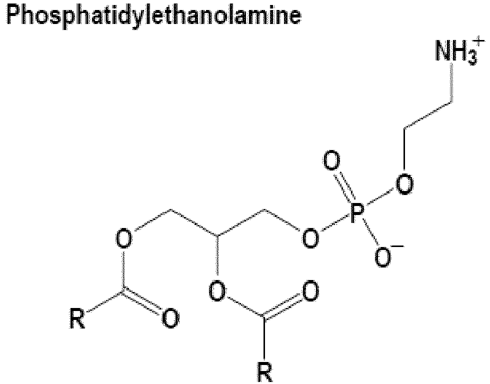

Dabei ist das Phosphorylcholin (PC) besonders bevorzugt.

Um das PC an die MMA/MAA zu koppeln wird das PC mit einer Vinylgruppe benutzt, welche durch radikalische Polymerisation an das MMA/MAA Präpolymer angebunden werden kann.

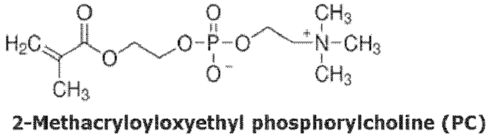

Zur Oberflächenmodifikation wird das in Figur 2 unten gezeigte Syntheseschema angewendet [Silikon aufquellen (Pfeil nach unten) Monomer zur gequollenen Matrix geben (Pfeil nach unten) oberflächlich mit Initiator und PC waschen (Pfeil nach unten) Polymerisation] .

### Synthesevorschrift:

Substrate aus Silikon Nusil® Med 45xx werden in eine Lösung aus MMA/MAA (2:1) gegeben. Dazu werden 100 ml MMA mit 50 ml MAA gemischt und die Substrate für 24 h bei Raumtemperatur gelagert und zum Quellen gebracht.
Nach dem Einlegen der Proben wird die Lösung 2 min mit Stickstoff begast, um Sauerstoff aus der Lösung zu entfernen.
Für die Weiterbehandlung wird 10 mg/ml 2,2'-Azobis(2-methylpropionamidin)dihydrochlorid (AIBN) in Wasser gelöst und 10 mg/ml 2-Methacryoxyethyl PC dazugegeben und ebenfalls bei Raumtemperatur gelöst.

Die Lösung wird 5 min mit Stickstoff entgast. Die gequollenen Silikonproben werden in diese wässrige Lösung gegeben und 1 bis 3 min gewaschen. Die Proben werden entnommen und 24 h bei 80 °C inkubiert.

In diesem Ausführungsbeispiel ist das Monomer zugleich das Quellungsmittel. Alternativ kann das Quellungsmittel auch Cyclohexan, Cycloheptan, N-Heptan oder allgemein ein Kohlenwasserstoff sein.

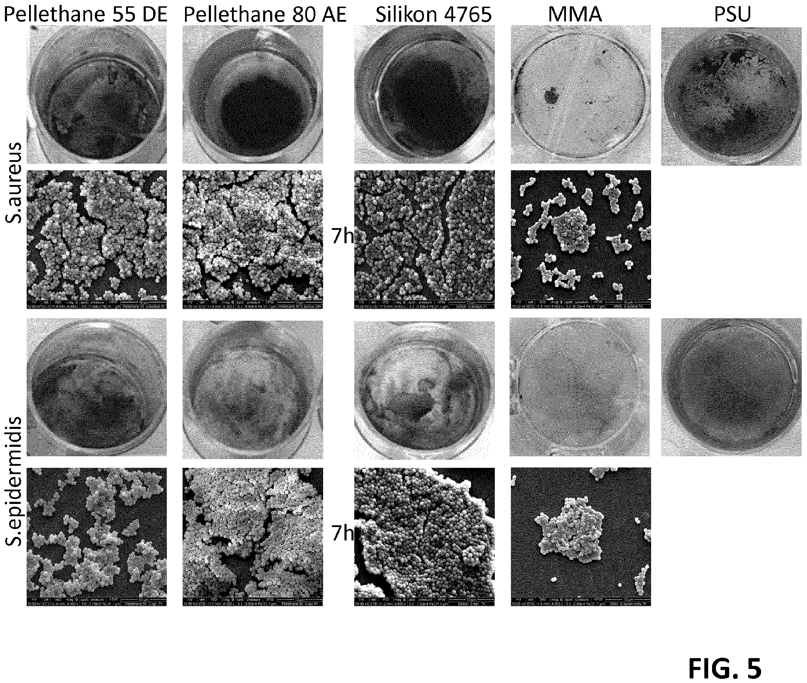

Das Ausführungsbeispiel erzeugt Oberflächen die sich als antimikrobiell herausgestellt haben (vgl. Figur 5).

In einem weiteren Ausführungsbeispiel wird die Silikonmatrix ebenfalls zum Quellen gebracht. Als Lösungsmittel werden neben Cyclohexan, Cycloheptan, N-Heptan oder allgemein einem Kohlenwasserstoff auch Dimethylacetamid und N-Methyl-2-pyrrolidon als besonders bevorzugte Lösungsmittel verwendet. In diesem Beispiel wird nicht ein Monomer sondern ein Polymer dazugegeben, um durch Diffusion ein IP-Netzwerk zu erzeugen. Als Polymere werden PESU und PSU verwendet.

Dazu wird die Silikonmatrix für 48 h im Lösungsmittel bei Raumtemperatur gequollen. Das Polymer wird als Feststoff dazugegeben und in Gegenwart der zu behandelnden Silikonproben im Lösungsmittel bei Raumtemperatur über 24 h gelöst. Die Silikonproben werden dann noch für weitere 24 h in dieser Lösung inkubiert und dann entnommen, von anhaftender Lösung befreit und bei 40 °C für 8 h vorgetrocknet und dann bei 40 °C und 0,01 Bar im Vakuum vom Restlösemittel befreit.

### Ausführungsbeispiel Elektrodenleitung

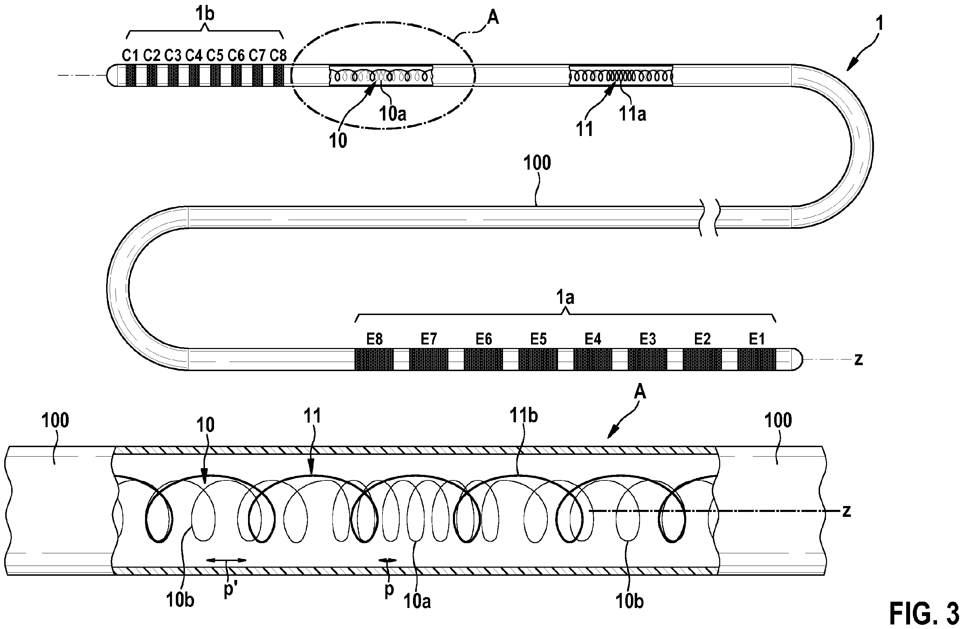

Eine Ausführungsform des erfindungsgemäßen Implantats ist in Figur 3 dargestellt, welche eine Elektrodenleitung 1 aufweist. Die Elektrodenleitung umfasst eine Vielzahl von Leitern 10, 11, welche sich longitudinal entlang der z-Achse der Elektrodenleitung 1 erstrecken. Die Leiter 10,11 sind hierbei in Form einer Helix um die Longitudinalachse z der Elektrodenleitung gewunden.

Die Elektrodenleitung 1 weist eine äußere (z. B. röhrenförmige) elektrische Isolierung 100 auf, die sich entlang der longitudinalen Achse z der Elektrodenleitung 1 erstreckt und die die Leiter 10,11 umschließt. Die elektrische Isolierung 100 weist hierbei den erfindungsgemäßen Werkstoff auf oder besteht im Wesentlichen daraus.

Die Leiter 10, 11 sind vorzugsweise als Kabel ausgebildet, welche eine Vielzahl von Drähten umfassen können, die beispielsweise Silber oder Tantal umfassen können oder im Wesentlichen daraus bestehen können.

In der in Figur 3 dargestellten Detailansicht des Bereichs A ist erkennbar, dass die eine Helix bildenden Leiter 10, 11 entlang der z-Achse mit unterschiedlicher Ganghöhe p, p' der Helix gewickelt sein können (vgl. Bezugszeichen 10a, 10b und 11b).

Die Elektrodenleitung 1 weist in ihrem distalen Bereich 1a Elektrodenpole E1 bis E8 zur Kontaktierung von Körpergewebe auf. In ihrem proximalen Bereich 1b verfügt die Elektrodenleitung weiterhin über Kontakte C1 bis C8, mit welchen die Elektrodenleitung an ein aktives Elektrisches Implantat wie einen implantierbaren Pulsgenerator - beispielsweise einen implantierbaren Herzschrittmacher oder einen implantierbaren Neurostimulator - oder einen ICD angeschlossen werden kann. Die Kontakte C1 bis C8 werden dabei mittels elektrischer Leiter 10, 11 jeweils mit den Elektrodenpolen E1 bis E8 verbunden. Hierfür kann die Elektrodenleitung mehr als zwei Leiter 10, 11, wie in Figur 3 schematisch eingezeichnet, aufweisen. Insbesondere kann die Elektrodenleitung zur Verbindung des Elektrodenpols E1 mit dem Kontakt C1 einen eigenen elektrischen Leiter aufweisen. Entsprechendes gilt auch für die anderen Elektrodenpol-Kontakt-Paare E2 und C2, E3 und C3 etc.

### Ausführungsbeispiel Elektrodenfixierhülse

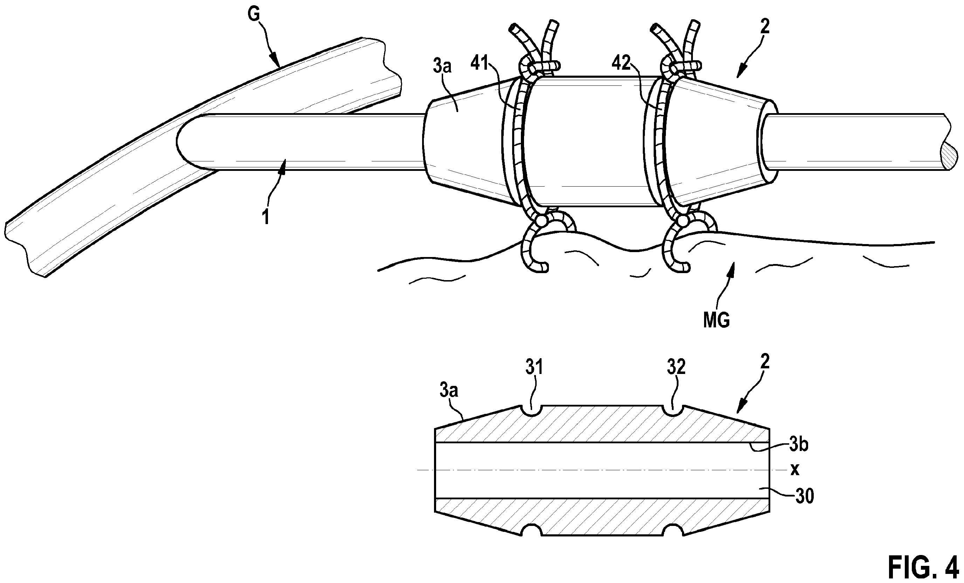

Eine andere Ausführungsform des erfindungsgemäßen Implantats betrifft eine Elektrodenfixierhülse 2, wie sie exemplarisch in Figur 4 dargestellt ist. Die Fixierhülse dient zum Fixieren eines längs erstreckten Elements 2, hier bspw. in Form einer implantierbaren Elektrodenleitung 1. Die Elektrodenleitung 1 weist einen längs erstreckten Leitungskörper auf, in dem in bekannter Weise zumindest ein - hier nicht gezeigter - elektrischer Leiter angeordnet ist, der insbesondere mit einem Elektrodenkontakt verbunden ist, der an einer Oberfläche des Leitungskörpers angeordnet sein kann, um Gewebe des Patienten zu kontaktieren. Bei dem Beispiel gemäß Figur 1 punktiert die Elektrodenleitung 1 ein Gefäß G des Patienten und ist über Fixierelemente 41, 42, die an der Fixierhülse 2 festgelegt sind mit Muskelgewebe MG des Patienten verbunden. Die Fixierelemente 41, 42 dienen weiterhin zum Komprimieren bzw. Einschnüren der Fixierhülse 2, derart, dass die Elektrodenleitung 1 in einem Lumen 30 der Hülse 3 festgeklemmt wird. Dabei liegt die Elektrodenleitung 1 an einer Innenseite 3a der Hülse 2 an. Die Fixierelemente 41, 42 sind zum Ausüben der jeweiligen Kraft in der jeweiligen Rille 31, 32 um die Hülse 2 herumgelegt, so dass mittels des jeweiligen Fixierelements 41, 42 eine Einschnürung der Hülse realisierbar ist, die eine Kompression der Hülse 3 zumindest im Bereich der jeweiligen Rille 31, 32 zur Folge hat, derart, dass die Elektrodenleitung 1 im Lumen 30 der Hülse 2 festgeklemmt wird. Erfindungsgemäß ist vorgesehen, dass die Elektrodenhülse und/oder die Elektrodenleitung den erfindungsgemäßen Werkstoff aufweist. Insbesondere ist vorgesehen, dass die Elektrodenleitung eine elektrische Isolierung aufweist, die den erfindungsgemäßen Werkstoff aufweist, insbesondere auf der äußeren Oberfläche, oder die elektrische Isolierung im Wesentlichen aus dem erfindungsgemäßen Werkstoff besteht. Weiterhin ist insbesondere vorgesehen, dass die Elektrodenfixierhülse im Wesentlichen aus dem erfindungsgemäßen Werkstoff gefertigt ist.

### Antimikrobielle Eigenschaften des erfindungsgemäßen Werkstoffes

In Figur 5 sind die Ergebnisse von Bewuchsversuchen mit verschiedenen handelsüblichen Materialien, wie etwa thermoplastische Polyurethan-Elastomere (Pellethan 55DB oder 80 AE), Silkone oder Silikone (Silikon 4765), und erfindungsgemäßen Werkstoffen (Silikon modifiziert mit MMA oder PSU). Die erfindungsgemäßen Werkstoffe weisen hierbei einen geringeren Bewuchs mit *S. aureus* oder *S. epidermidis* auf.

## Patentansprüche

1. Werkstoff umfassend ein Silikon, **dadurch gekennzeichnet, dass** ein Polymer an der Oberfläche des Silikons angeordnet ist, wobei das Polymer durch eine höhere Abriebfestigkeit als das Silikon charakterisiert ist, und das Polymer über nicht-kovalente Bindungen an der Oberfläche des Silikons befestigt ist.

2. Werkstoff nach Anspruch 1, **dadurch gekennzeichnet, dass** das Polymer ein Copolymer aus zwei oder mehreren Monomeren ist, wobei insbesondere ein oder mehrere Monomere aus der Gruppe ausgewählt sind, die:
- ein aromatisches Diphenol, insbesondere Bisphenol A oder 1,4-Dihydroxybenzen, und ein Bis(halophenyl)sulfon, insbesondere 4,4-Dichlordiphenylsulfon,
- Vinylidenfluorid, und
- ein Acrylat, ein Methacrylat. und/oder ein Methylmethacrylat umfasst.

3. Werkstoff nach Anspruch 1 oder 2, **dadurch gekennzeichnet, dass** das Polymer ein Polyvinylidenfluorid, ein Polyarylsulfon, insbesondere Polysulfon, Polyethersulfon, oder Polyphenylensulfon, oder ein Polyacrylat, ein Polymethylacrylat oder ein Polymethylmethacrylat oder Polytetrafluorethylen (PTFE), Ethylen-Tetrafluorethylen (ETFE), Polyamid, Polyetheretherketon (PEEK) ist.

4. Werkstoff nach einem der vorherigen Ansprüche, **dadurch gekennzeichnet, dass** das Polymer durch Verschlaufung der Ketten des Silikons und der Ketten des Polymers an der Oberfläche des Silikons befestigt ist.

5. Werkstoff nach einem der vorherigen Ansprüche, **dadurch gekennzeichnet, dass** das Silikon ein Methyl-Silikon, insbesondere ein Polydimethylsiloxan, ein Vinyl-Methyl-Silikon, ein Phenyl-Vinyl-Methyl-Silikon, ein phenylmodifiziertes Silikon, ein Fluoroalkyl-Silikon oder ein Fluor-Vinyl-Methyl-Silikon ist.

6. Werkstoff nach einem der vorherigen Ansprüche, **dadurch gekennzeichnet, dass** das Polymer ein Copolymer aus
a. einem Acrlyat, einem Methacrylat und/oder einem Methylmethacrylat; und
b. einer zwitterionischen Verbindung ausgewählt aus Phosphorylcholin, Sulfobetain, Carboxybetain, Sulfopyridiumbetain, Cystein, Phosphatidylethanolamin oder ein Sulfobetainsiloxan, vorzugsweise umfassend Methyl- oder Ethylgruppen, ist.

7. Medizinisches Implantat aufweisend einen Werkstoff gemäß einem der vorherigen Ansprüche.

8. Medizinisches Implantat nach Anspruch 7, **dadurch gekennzeichnet, dass** das medizinische Implantat eine Elektrodenleitung umfasst.

9. Medizinisches Implantat nach Anspruch 8, **dadurch gekennzeichnet, dass** die Elektrodenleitung eine elektrisch isolierende Ummantelung aufweist, die den Werkstoff nach einem der Ansprüche 1 bis 6 aufweist oder im Wesentlichen daraus besteht.

10. Medizinisches Implantat nach Anspruch 8 oder 9, **dadurch gekennzeichnet, dass** das medizinische Implantat eine stromerzeugende bzw. stromgebende Komponente und/oder eine stromerfassende Komponente umfasst, welche mit der Elektrodenleitung verbunden ist, wobei das medizinische Implantat insbesondere ein Herzschrittmacher, ein Kardioverter-Defibrillator oder ein Neurostimulator ist.

11. Medizinisches Implantat nach Anspruch 8 oder 9, **dadurch gekennzeichnet, dass** das Implantat eine Fixierhülse zum Fixieren einer implantierbaren Elektrodenleitung umfasst, wobei die Fixierhülse einen Werkstoff gemäß einem der Ansprüche 1 bis 6 aufweist oder im Wesentlichen daraus besteht.

12. Verfahren zur Herstellung eines Werkstoffes nach einem der Ansprüche 1 bis 6, umfassend die Schritte:
- Bereitstellen eines Silikons, und
- Anordnen eines Polymers auf die Oberfläche des Silikons, wobei das Polymer durch eine höhere Abriebfestigkeit als das Silikon gekennzeichnet ist, und das Polymer über nicht-kovalente Bindungen an der Oberfläche des Silikons befestigt wird.

13. Verfahren nach Anspruch 12, **dadurch gekennzeichnet, dass** das Anordnen des Polymers auf die Oberfläche des Silikons folgende Schritte umfasst:
- Lösen des Polymers in einem geeigneten Lösungsmittel unter Erhalt einer Polymerlösung;
- Auftragen der Polymerlösung auf das Silikon, vorzugsweise durch Aufsprühen oder Eintauchen; und
- Trocknen des Silikons bei einer Temperatur im Bereich von Raumtemperatur bis etwa 300 °C, vorzugsweise bei Temperaturen von 180 °C bis 260 °C und weiter bevorzugt bei etwa 220 °C.

14. Verfahren nach Anspruch 12, **dadurch gekennzeichnet, dass** das Anordnen des Polymers auf die Oberfläche des Silikons folgende Schritte umfasst:
- Quellen des Silikons in einem organischen Lösungsmittel unter Erhalt eines gequollenen Silikons,
- In Kontakt bringen des gequollenen Silikons mit dem Polymer, wobei das Polymer gelöst in einem geeigneten Lösungsmittel vorliegt, oder
- In Kontakt bringen des gequollenen Silikons und einem oder mehreren Vorläufern des Polymers, wobei der eine/die mehreren Vorläufer des Polymers gelöst in einem geeigneten Lösungsmittel vorliegen, und Umsetzen des einen oder der mehreren Vorläufer zum Polymer, und
- Trocknen des Silikons.

15. Verfahren nach Anspruch 14, **dadurch gekennzeichnet, dass** das organische Lösungsmittel Toluol, Chloroform, Dichlormethan, Dimethylacetamind, Dimethylformamid, Dimethylsulfoxid, Kresol, -ortho-Dichlorbenzol, Sulfolan, Tetrahydrofuran., Trichlorethylen oder N-Methyl-2-pyrrolidon, insbesondere N-Methyl-2-pyrrolidon, vorzugsweise in Kombination mit n-Heptan, oder eine Methylmethacrylat- oder Methacrylatlösung ist.
